# EUROPÄISCHE PATENTSCHRIFT

(11) **EP 4 507 620 B1**
(45) Veröffentlichungstag und Bekanntmachung des Hinweises auf die Patenterteilung: **10.06.2026**
(21) Anmeldenummer: 23717070.9
(22) Anmeldetag: 30.03.2023
(51) Int. Cl.: A61C 7/08, A61C 7/10, A61C 19/04, A61N 1/00, A61N 5/00

(54) **MYOFUNKTIONELLER ALIGNER**
MYOFUNCTIONAL ALIGNER
ALIGNEUR MYOFONCTIONNEL

(30) Priorität: 11.04.2022 DE 102022108777
(43) Veröffentlichungstag der Anmeldung: 19.02.2025
(73) Patentinhaber: CODONIS AG, 4132 Muttenz (CH)
(72) Erfinder: CIRILLO, Fabio, 4466 Ormalingen (CH); STÜBINGER, Stefan, 4102 Binningen (CH)
(74) Vertreter: Kleine, Hubertus
(86) Internationale Anmeldenummer: PCT/EP2023/058354
(87) Internationale Veröffentlichungsnummer: WO 2023/198473

(56) Entgegenhaltungen:
- EP-A1- 3 838 247
- DE-A1- 102008 041 989
- US-A1- 2007 163 603
- US-A1- 2010 311 008
- US-A1- 2015 182 305
- US-A1- 2016 081 769
- US-A1- 2017 252 140
- US-A1- 2020 215 384

## Beschreibung

### Technisches Gebiet

Die vorliegende Erfindung betrifft einen myofunktionellen Aligner als Behandlungsgerät in der Zahnmedizin.

### Stand der Technik

Aus dem Stand der Technik sind zahnmedizinische Ausrichtungsvorrichtungen in Form von speziellen Zahnschienen, sogenannte Aligner, bekannt. Diese Aligner und die damit verbundene Aligner-Therapie. Immer dort, wo feste oder lösbare Klammern und Zahnspangen aus ästhetischen Gründen oder aus Gründen der Mundhygiene abgelehnt werden, kommen diese zumeist transparenten wandstärkendünnen Zahnschienen zum Einsatz. Entsprechende Aligner sollten, anders als lösbare Zahnspangen und Klammern, alle 1-4 Wochen gewechselt werden.

Aligner behandeln Fehl- und Engstände von Zähnen im Anfangsstadium, vorzugsweise im Bereich der Frontzähne. Auch zur Schließung von Lücken, z.B. nach kieferorthopädischen Eingriffen, kann ein Aligner eingesetzt werden.

Besonders hervorzuheben ist auch die Akzeptanz von Alignern gegenüber Zahnspangen, Brackets, Bögen oder anderen Methoden unter Jugendlichen, da die vorgenannten alternativen Varianten der Zahnkorrektur einen starken Eingriff in das optische Erscheinungsbild des Jugendlichen und/oder in dessen Aussprache haben.

Relevanter Stand der Technik findet sich in der US 2016/0081769 A1, der US2020/0215384A1, der US 2016/287481 A1, der US 2015/297464 A1, der WO 2016/192985 A1 und der WO 2015/050813 A1.

Die Aufgabe der vorliegenden Erfindung liegt in der Funktionserweiterung eines Aligners um eine zusätzliche myofunktionelle Stimulation.

### Offenbarung der Erfindung

Die vorliegende Erfindung löst die vorliegende Aufgabe durch das Bereitstellen eines Aligners mit den Merkmalen des Anspruchs 1.

Ein erfindungsgemäßer Aligner dient in erster Linie dem Ausrichten von Zähnen eines Gebisses. Dabei weist der Aligner (zahnmedizinischer Fachbegriff - übersetzt "Ausrichter") in bekannter Weise eine Grundform zur Vorgabe einer bevorzugten Zahnstellung auf. Diese Grundform weist eine U-Form auf, welche einen zur U-Form-komplementären bogenförmigen Innenraum begrenzt. Die Grundform kann materialbedingt eine unbeabsichtigte mechanische Zungenstimulanz darstellen, so dass die Zunge angeregt wird den Aligner im vorderen Bereich zu berühren. Die Folge kann eine falsche Aussprache, z.B. Lispeln und dergleichen sein, welche nach der zahnmedizinischen Anwendung, insbesondere bei Kindern, eine logopädische Behandlung zur Folge haben.

Der erfindungsgemäße Aligner weist daher einen von der Grundform in Richtung des Innenraums gerichteten Zungen-Ankerpunkt zu dessen Positionierung im bestimmungsgemäßen Zustand nahe dem Gaumendach einer Mundhöhle auf.

Der Zungen-Ankerpunkt soll die Zunge, insbesondere die Zungenspitze, dahingehend stimulieren, dass die Zunge von der Grundform weg hin zum Gaumendach geführt wird. Dadurch wird die Zungenbewegung stärker gefördert.

Vorteilhafte Ausgestaltungen der Erfindung sind Gegenstand der Unteransprüche.

Der Aligner, insbesondere die Grundform, kann vorteilhaft eine u-förmige, insbesondere geschlossenwandige, Innenwandung zur lingualen Bedeckung einer Zahnreihe (Oberkiefer oder Unterkiefer) aufweisen.

Weiterhin kann der Aligner, insbesondere die Grundform, auch in gleicher Weise eine Außenwandung zur labialen Überdeckung einer Zahnreihe aufweisen.

Der Aligner, insbesondere die Grundform, kann vorteilhaft zur optimaleren Ausrichtung Zahneinzelkonturen mit einer vordefinierten Zahnstellung und mit zumindest teilweise buccalen Überdeckungsbereichen von einzelnen Zähnen einer Zahnreihe aufweisen.

Der Zungen-Ankerpunkt ist als Teil eines Vorsprungs ausgebildet, welcher von der Innenwandung in Richtung des Innenraums hervorsteht.

Alternativ oder zusätzlich ist der Zungen-Ankerpunkt als Teil einer Überbrückung ausgebildet, welche von dem ersten Schenkel der U-Form zum zweiten Schenkel der U-Form verläuft und somit den Innenraum überbrückt. Der Zungen-Ankerpunkt kann einen runden Körper, insbesondere eine Kugel und/oder einen Ovoid, aufweisen.

Der Zungen-Ankerpunkt kann eine gegenüber benachbarten Bereichen differente Oberflächentextur, insbesondere eine Rillung, Riffelung und/oder Noppung aufweisen. Die benachbarten Bereiche sind insbesondere ebenfalls Teil der Überbrückung oder des Vorsprungs.

Der Zungen-Ankerpunkt weist eine geometrische Figur auf, z.B. eine Ausbuchtung oder Nase, welche gegenüber einem Verbindungsbereich des Vorsprungs und/oder der Überbrückung mit der Grundform zumindest teilweise hervorsteht.

Weiterhin kann der Vorsprung oder die Überbrückung gegenüber der Grundform lösbar, vorzugsweise austauschbar, insbesondere verrastbar, angeordnet sein.

Erfindungsgemäß ist der Zungen-Ankerpunkt gegenüber dem Vorsprung oder der Überbrückung lösbar und austauschbar, insbesondere verrastbar, angeordnet.

Der Zungen-Ankerpunkt kann vorzugsweise gegenüber benachbarten Bereichen der Überbrückung oder des Vorsprungs eine differente Oberflächenhaptik, vorzugsweise eine verschiedene Rauhigkeit und/oder Weichheit des Materials, aufweisen.

Weiterhin kann der Zungen-Ankerpunkt eine in Speichel zumindest teilweise lösliche chemische Verbindung, insbesondere einen Geschmacksstoff oder einen Wirkstoff, aufweisen. Hier kann eine andere als die mechanische Stimulanz oder eine zusätzliche Stimulanz eingesetzt werden. Dabei ist die Austauschbarkeit des Zungen-Ankerpunkts oder aber der gesamten Überbrückung oder des Vorsprungs besonders von Vorteil. Auch ein Geruchsstoff z.B. zur Verbesserung des Atems ist denkbar.

Der Zungen-Ankerpunkt kann vorteilhaft eine Beschichtung aufweisen, welche auf einem Trägermaterial aufgebracht ist.

Vorteilhaft können im Bereich des Zungen-Ankerpunkts Öffnungen, insbesondere Schlitze, vorgesehen sein.

Im Bereich des Zungen-Ankerpunkt kann ein Material aus einer anderen chemischen Zusammensetzung angeordnet sein oder aber der Zungen-Ankerpunkt kann bereichsweise oder vollständig aus einer anderen Materialklasse bestehen, als die benachbarten Bereiche des Vorsprungs und/oder der Überbrückung. Eine andere Materialklasse ist z.B. Metall anstelle von Kunststoff.

Der Zungen-Ankerpunkt kann vorteilhaft bewegliche Elemente aufweisen, welche durch die Zunge bewegbar sind.

Der Aligner, insbesondere die Außenwandung zwischen Zahnreihe und Lippen, kann ein Schildsegment zum bereichsweisen Überdecken einer Zahnreihe, insbesondere der Zahnreihen des Ober- und Unterkiefers, aufweisen, wobei das Schildsegment auf der zahnabgewandten Seite zumindest einen Vorsprung, insbesondere eine Anordnung mehrerer Vorsprünge, zur Lippenstimulation aufweist.

Die Vorsprünge können vorzugsweise eine Höhe von zumindest 0.1 mm, vorzugsweise grösser 0.5 mm aufweisen. Ebenfalls vorzugsweise können die Vorsprünge zylindrisch, kalotten- oder kegelstumpfförmig ausgebildet sein.

Zudem kann der Aligner Sensoren zur Ermittlung deiner physikalischen und/oder chemischen Größe im Speichel und/oder im Mundraum aufweisen, wobei die Sensoren besonders bevorzugt Teil des Vorsprungs oder der Überbrückung sind.

Auch die Vorsprünge des Schildsegments können zur Überwachung des fortschreitenden Trainingserfolgs und/oder des Gesundheitszustands im Mund Sensorelemente aufweisen. Zur Unterstützung des Trainings kann das Behandlungsgerät zudem Aktuatoren aufweisen

Weiterhin kann der Aligner Aktuatoren, insbesondere Elektroden, zur Erzeugung und/oder Übermittlung von Stimulanzsignalen aufweisen, wobei die Aktuatoren besonders bevorzugt Teil des Vorsprungs oder der Überbrückung sind.

Bevorzugt kann der Aligner die Möglichkeit einer modularen Anpassung an verschieden große Ober- und Unterkiefersituationen auf.

Der Aligner, insbesondere die Überbrückung oder der Vorsprung können alternativ oder zusätzlich einen Wirkstoff und/oder einen Geschmacksstoff und/oder einen Indikatorstoff und/oder einen Biomarker, aufweisen.

Der Aligner kann zudem vorteilhaft einen teleskopartigen Auszug zur Adaptierung des Aligners an die Größe eines Gebisses aufweisen.

Der Aligner kann alternativ auch einstückig, insbesondere monolithisch, ausgebildet sein.

Alternativ kann Aligner modular aufgebaut sein. Besonders bevorzugt sind dabei die Überbrückung und/oder der Vorsprung mit dem Zungenankerpunkt austauschbar als Wechselmodule an der Grundform angeordnet. Dadurch kann die Intensität der Stimulanz eingestellt werden. Jeder Mensch reagiert auf mechanischen Reiz oder andere Formen der Stimulanz unterschiedlich stark. Durch die Austauschbarkeit ist der Aligner individuell einstellbar.

### Kurze Beschreibung der Zeichnungen

Nachfolgend wird die Erfindung anhand von Ausführungsbeispielen unter Bezug auf die Zeichnung näher beschrieben, wobei auch weitere vorteilhafte Varianten und Ausgestaltungen diskutiert werden. Es sei betont, dass die nachfolgend diskutierten Ausführungsbeispiele die Erfindung nicht abschließend beschreiben sollen, sondern dass auch nicht dargestellte Varianten und Äquivalente realisierbar sind und unter die Ansprüche fallen. Es zeigt:
- Fig. 1: Prinzipdarstellung einer ersten Variante eines erfindungsgemäßen Aligners;
- Fig. 2: Prinzipdarstellung einer zweiten Variante eines erfindungsgemäßen Aligners;
- Fig. 3: Darstellung des Aligners der Fig. 2 mit einer Überbrückung im demontierten Zustand;
- Fig. 4 a-u: Varianten für die Überbrückung der Fig. 2 und 3 und
- Fig. 5: Variante zur Ausgestaltung einer Außenwandung 5 im Rahmen der vorliegenden Erfindung.

### Ausführungsform(en) der Erfindung

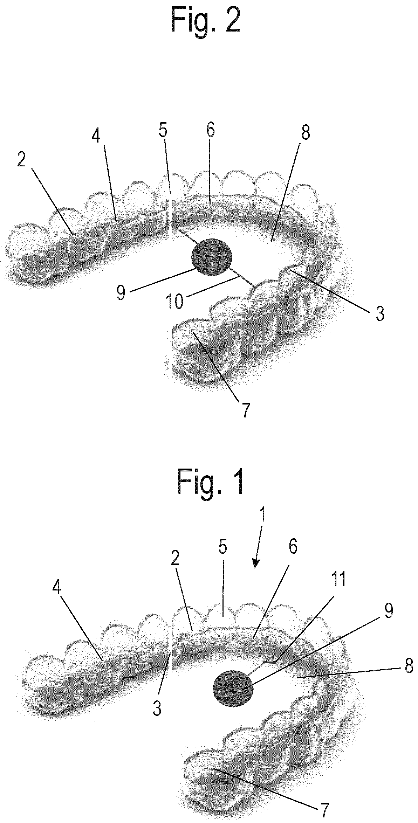

Fig. 1 zeigt eine schematische Darstellung eines Aligners 1 zur Ausrichtung von Zähnen. Der Aligner 1 weist in der Draufsicht eine U-förmige Grundform 3 auf, mit Zahneinzelkonturen 2 zur besseren Einfassung von Einzelzähnen.

Die Schnittansicht senkrecht zur Abfolgerichtung einer Zahnreihe durch den Aligner ist ebenfalls U-Förmig, so dass der Aligner eine Schalenform 7 zur Einfassung der Zahnreihe aufweist.

Dabei weist eine Zahneinzelkontur 2 Teilbereiche auf und ragt teilweise von den buccalen und lingualen Überdeckungsbereichen der Zähne in die Zahnzwischenräume 4 hinein. Dabei ist bildet die Außenfläche des U-Schenkels der Grundform eine geschlossene Außenwandung 5 und eine geschlossenen Innenwandung 6 über den gesamten Verlauf der U-Form des Grundkörpers 3 auf. Die Außenwandung 5 und die Innenwandung 6 begrenzen den Bereich zur Aufnahme der Zähne der u-förmigen Schalenform 7. Die Zahneinzelkonturen 2 sind je nach Zahnart, z.B. Eckzahn, Schneidezahn und/oder Backenzahn, ausgebildet.

Die Innenwandung 6 der Schalenform 7 definiert aufgrund ihrer U-Form zu entständig jenseits der Backenzähne und/oder Weisheitszähne offenen Innenraum 8 in welchem typischerweise die Zunge des Nutzers positioniert ist.

Gemäß einer ersten Variante der Erfindung erstreckt sich von der Innenwandung 6 in diesen Innenraum 8 eine Überbrückung 10, insbesondere mit einer Ausformung, welche nachfolgend als Zungen-Ankerpunkt 9 bezeichnet wird.

Dadurch berücksichtigt der Aligner eine sogenannte Myofunktion, mit welcher zusätzlich zur Zahnkorrektur ein Lispeln, Mundatmung, falsches oder fehlendes Schluckmuster, Hypersalivation, Muskelspasmen und/oder Muskelverspannung, insuffizienter Lippen- und/oder Mundschluss oder andere Störungen verhindert werden.

Typischerweise liegt bei einem üblichen Gebiss das vordere Zungendrittel hinter den oberen Frontzähnen flächig am Gaumendach an. An dieser Stelle soll der Zungen-Ankerpunkt 9 positioniert werden. Entsprechend ist der Abstand des Zungen-Ankerpunkts 9 gegenüber der Innenwandung 6 auszugestalten.

Der Zunken-Ankerpunkt regt an, dass der Patient bewusst die Zunge dort platziert wird. Dies betrifft Patienten, die eine Zahnkorrektur bekommen sollen, und anderseits auch für Patienten die eine logopädische oder myofunktionelle Therapie brauchen.

Der Aligner und die Schalenform kann individuell nach Abformung und/oder scan, z.B. durch den Zahnarzt vom Zahntechniker, hergestellt werden.

Zusätzlich zu dem Material des Zungen-Fix/Ankerpunkts 9 am Gaumendach, können weitere Elemente vorgesehen sein, so z.B. Sensoren, eine Modularität im Aufbau, ein Drug-release-System und/oder eine Stimulation durch Noppen etc. an der Außenwandung 5 des Aligners.

Die Verwendung von Sensorelementen hat den besonderen Effekt, dass z.B. bei Anwendung von Biosensoren ein Entzündungsstatus durch die Konzentration einzelner Speichelinhaltsstoffe erfasst werden kann. Eine Leitfähigkeitsmessung und/oder andere physikalische Messungen, wie z.B. eine Temperaturmessung, können die Mundraumtemperatur, den Feuchtigkeitszustand im Zwischenraum und ggf. den Umfang der Durchblutung der Lippen ermittelt werden. Zudem können mit Drucksensoren z.B. der Zungenanpressdruck oder der Muskeltonus der Kau- und Gesichtsmuskultur gemessen werden.

Elektroden können Stimulanzsignale, wie z.B. Reizstrom, zur Förderung der Durchblutung und der Muskulatur aussenden. Ebenfalls sind Aktoren zur Massage von Lippen, Muskulatur oder allgemein Gewebe einsetzbar.

Im Fall von elektrisch-betriebenen Sensorelementen oder Elektrode kann innerhalb des Aligners eine Energiespeichereinheit und/oder eine Regel und/oder Auswerteeinheit angeordnet sein, welche vorzugsweise vom Material des Aligners ummantelt ist und damit nicht mit Speichel in Berührung kommt. Es kann aufgrund der hohen mechanischen Belastung von Elektronikbauteilen bei bestimmungsgemäßer Anwendung des Aligners auch eine mehrschichtige Ummantelung aus Material unterschiedlicher Härten vorgesehen sein.

Der Aligner 1 kann an verschiedene Größen für verschiedene Gebisse angepasst werden, i.e. Kinderzähne, Wechselgebiss (von Milch- zu bleibenden Zähnen), bleibende Zähne und Prothesen. Dabei kann auch lediglich die Länge der Schalenform 7 nach Distal angepasst werden.

Da solche Sensoren, aktive Sensoren mit Strombedarf sind, kann der Basiskörper weiter eine Energiequelle, Energiezelle oder Energie-"harvesting"-Device beinhalten.

Vorzugsweise sind das passive, elektrochemische Zellen, Thermo-elektrische Elemente und/oder induktions-geladene Akkumulatoren oder kapazitive Elektrospeicher. Gerade bei Elementen, die in der Art von Intarsien am oder im Aligner1 angeordnet sind, können diese, nach Bedarf, ausgewechselt und in den Aligner eingesetzt werden.

Zusätzlich können die Sensoren direkt mit geeigneten externen Geräten gekoppelt werden um mittels Sensorik eine Steuerung, Schallwellenleitung und/oder Programme anzusprechen.

Zusätzlich kann die externe Basisstation zur Aufbewahrung des Aligners dienen, als auch zum Aufladen des Aligners, oder/und auslesen der Sensorik. Weiter kann die externe Basisstation die Daten an einen zentralen Speicher übermitteln, e.g. Cloud-Speicher. oder/und Relais-Station, wie Smartphones. Vorzugweise dient die externe Basisstation als Aufbewahrungsbox, Reinigungsbox und/oder Akkustation während Reisen.

Fig. 2 zeigt eine zweite schematische Variante einer erfindungsgemäßen Ausführungsvariante der Erfindung. Dabei werden identische Bauteile und Bereiche der Variante der Fig. 1 mit gleichen Bezugszeichen versehen. Die Variante der Fig. 2 weist einen Vorsprung 11 anstelle einer Überbrückung und einer ansonsten identisch-ausgebildeten u-förmigen Grundform 3. Der Vorsprung 11 dient ebenfalls einer Positionierung eines Zungen-Ankerpunkts 9 im Bereich eines Gaumendachs mittig im Innenraum 8 der U-Form der Grundform.

Dieser Innenraum 8 wird im bestimmungsgemäßen Einsatz bei geschlossenem Mund durch den Grundform 3, der benachbarten Zahnreihe oder einem benachbarten Aligner, sowie dem Gaumendach und dem Mundboden der Mundhöhle gebildet und weist in Richtung des Gaumenzäpfchens eine Öffnung auf.

Der besagte Zungen-Ankerpunkt 9 kann als individueller flächiger Fix-/Ankerpunkt für die Zunge ausgebildet sein, welcher vorzugsweise einem Sensor, einem Saugnapf, Noppen oder einer anderen Oberflächentextur, einer Zungenablage, einem Zungenstimulanzpunkt, z.B. einer mechanischen und/oder gustatorischen Stimulanz versehen sein kann.

Der Aligner kann zudem modular erweiterbar sein für z.B. für die Aufnahme von Schnarcherschienen-Zusätzen, zur Wirkstofffreigabe oder auch Patrizen-Matrizen-System zur Aufnahme von einem kleinen Gummiring der auf Zunge platziert wird und, vorzugsweise im Bereich des Zungen-Ankerpunkts 9, angedockt werden muss.

Durch den Zungen-Ankerpunkt wird insbesondere für das vordere Drittel der Zunge, insbesondere ab der Incisalpapille ein flächenhafter Kontakt mit dem Gaumen erreicht, wodurch die Nasenatmung stimuliert wird.

Die Lippen sind dabei vorzugsweise in lockerem Kontakt mit dem Aligner. Der Kinnmuskel kann sich entspannen und die Zahnreihen befinden sich in Ruheschwebe. Insgesamt ist damit die Ruhelage der Zunge optimal.

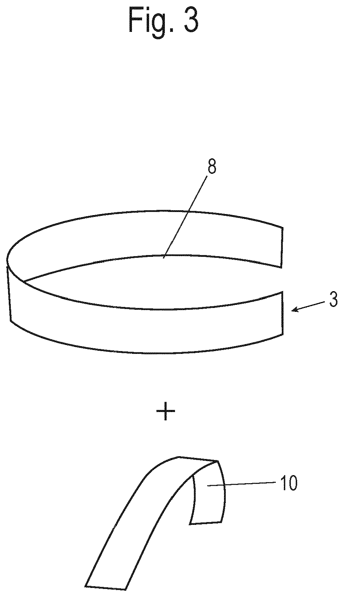

Die Überbrückung 10 der Fig. 1 ist vorteilhaft analog zu der in Fig. 3 dargestellten Variante bogenförmig in Richtung des Gaumendaches ausgebildet, so dass eine weitergehende Annäherung des Zungen-Ankerpunkts 9 in Richtung des Gaumendaches erreicht wird.

Die Überbrückung 10 ist in Fig. 3 lösbar, insbesondere austauschbar, dargestellt. Dies kann über eine beliebige Steck-, Klemm- oder Rastverbindung oder auf andere Art, z.B. durch Einsatz mechanischer Verbindungsmittel wie z.B. Schrauben, erreicht werden.

Dabei sind allerdings Rastverbindungen bevorzugt, da ein Ablösen einzelner Elemente des Aligners die Gefahr des Verschluckens, insbesondere beim Schlafen, hervorrufen kann.

Mechanische Verbindungsmittel wiederum bedürfen oftmals einer gewissen Materialdicke des Materials im Verbindungsbereich und erhöhen die Teileanzahl und damit die Komplexität des Aligners.

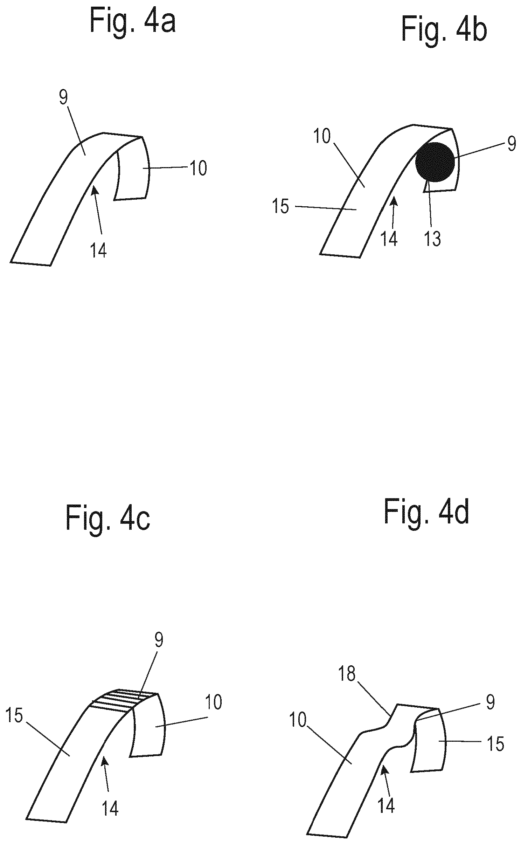

Fig. 4a zeigt zunächst eine Überbrückung 10, welche im oberen Materialbereich bereits den Zungen-Ankerpunkt 9 beinhaltet. Dabei ist das Material der Überbrückung über die gesamte Länge der Überbrückung einheitlich. Der Zungen-Ankerpunkt unterscheidet sich insofern nur durch seine Position, allerdings nicht durch seine physikalischen oder chemischen Eigenschaften von den benachbarten Gebieten der Überbrückung, durch welche seiner Fixierung am Grundkörper des Aligners dienen.

Fig. 4b zeigt eine Überbrückung mit einer Kugel 13 oder einem Ovoid, welcher auf der konkaven Seite 14 der Überbrückung 10, vorzugsweise mittig, angeordnet ist. Es kann auch eine Abfolge mehrerer dieser Kugeln und/oder Ovoide entlang der konkaven Seite 14 der Überbrückung angeordnet sein. Die Kugel 13 oder der Ovoid oder die Abfolge mehrerer Kugeln und/oder Ovoide können zudem erfindungsgemäß austauschbar, z.B. durch einen Rastverschluss oder dergleichen, an der Überbrückung angeordnet sein. Diese Varianten weisen gegenüber der Variante der Fig. 4a eine deutlich stärkere mechanische Stimulanz der Zunge auf.

Die Verbindung der Kugel 13 oder des Ovoiden oder der vorgenannten Abfolge mehrerer dieser Elemente mit der Überbrückung 10 kann zudem derart erfolgen, dass das jeweilige Element mechanisch beweglich ist. Hierfür kann entweder eine Fassung vorgesehen sein, welche die Kugel oder den Ovoiden bereichsweise einfasst. Gleiches ist auch für die Abfolge an Elementen möglich, ähnlich eines bogenförmig-ausgebildeten Wälzlagerkäfigs. Alternativ kann der Ovoid oder die Kugel auch mit einem zentralen Stift versehen sein, welcher senkrecht an der Überbrückung festgelegt ist und an welchem die Kugel oder der Ovoid rotierbar festgelegt ist.

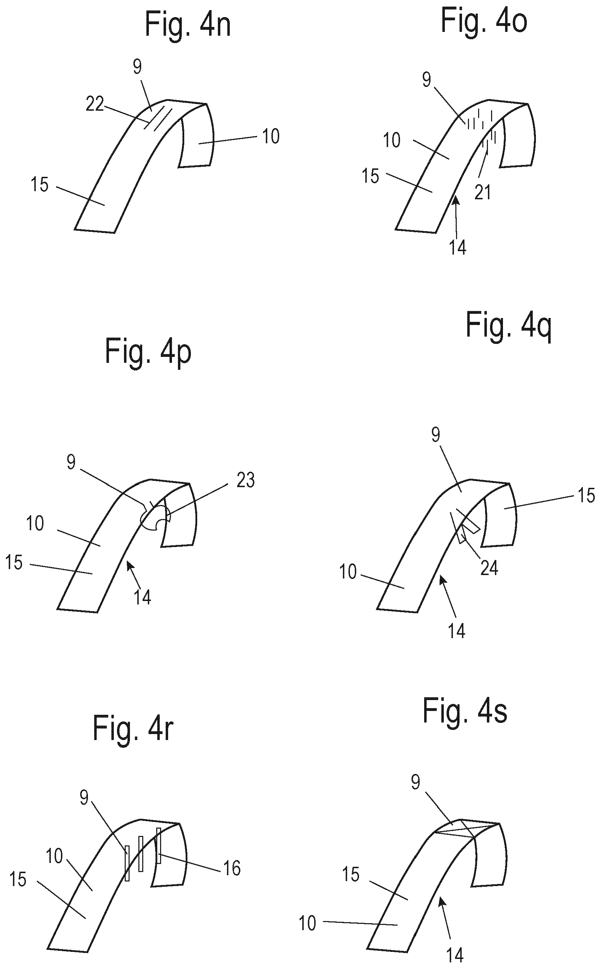

Fig. 4c weist eine Variante auf, in welcher der Zungen-Ankerpunkt 9 gegenüber benachbarten Bereichen 15 der Überbrückung 10 eine differente Oberflächentextur, in Form einer Riffelung aufweist. Alternativ ist beispielsweise auch eine Rillung und/oder Noppung möglich. Auch eine Anordnung von faden- oder streifenartigen abstehenden Elementen 16 (siehe Fig. 4o), Elemente mit Fasertextur und/oder flexible Nadeln 17, z.B. aus Gummi, (siehe Fig. 4r) oder dergleichen sind als Zungen-Ankerpunkt 9 möglich und erhöhen die mechanische Stimulanz der Zunge.

Fig. 4d zeigt eine Variante in welcher die Form der Überbrückung 10 selbst gegenüber den benachbarten Bereichen 15 geändert wurde. So ragt in die konkave Seite 14 der Überbrückung 10 zusätzlich eine konkave Ausbuchtung bzw. Nase 18 hinein. Diese sorgt für eine Erhöhung der mechanischen Stimulanz der Zunge.

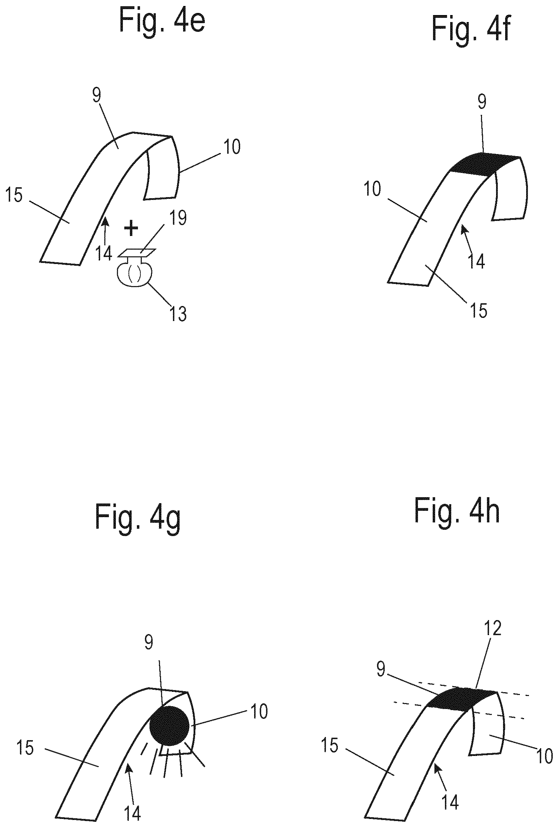

Fig. 4e zeigt eine weitere erfindungsgemäße Variante einer lösbaren Verbindung einer Kugel 13 mit der Überbrückung 10 z.B. durch einen Sockel 19. Eine komplementäre Aufnahmeöffnung mit randseitigen Führungsschlitzen in der Überbrückung 10 ist in Fig. 4e zwar nicht dargestellt, kann allerdings ohne Weiteres logisch ergänzt werden, so dass der Sockel seitlich in die Überbrückung 10 eingesteckt werden kann. Optional können die Führungsschlitze auch noch Rastnasen aufweisen, so dass ein seitliches Verschieben entgegen der Einführrichtung bei Erreichen der Endposition für die Kugel nicht ohne Betätigung des Rastmechanismus zur Entrastung nicht mehr möglich ist. Gleiches gilt selbstverständlich auch für die Anbindung anderer geometrischer Objekte, z.B. einem Ovoid.

Fig. 4f zeigt eine Variante, bei welcher der Zungen-Ankerpunkt 9 gegenüber benachbarten Bereichen 15 der Überbrückung 10, eine differente Oberflächenhaptik, vorzugsweise eine verschiedene Rauhigkeit und/oder Weichheit des Materials, aufweist. Dabei kann es sich um eine Oberflächentextur mit höherer Rauhigkeit im Bereich des Zungen-Ankerpunkts 9 handeln aber auch um eine besonders glatte Oberfläche, also mit geringerer Rauhigkeit im Bereich des Zungen-Ankerpunkts 9 oder aber auch um ein weicheres Material im Bereich des Zungen-Ankerpunkts, z.B. ein Knistermaterial, z.B. eine Knister- oder Raschelfolie oder um ein Blistermaterial oder dergleichen.

Fig. 4g zeigt eine Variante, bei welcher der Zungen-Ankerpunkt 9 zusätzlich oder alternativ zur mechanischen Stimulanz noch eine gustatorische Komponente freisetzt, z.B. einen Süßstoff, einen Bitterstoff, einen sauer-schmeckenden Stoff oder dergleichen. Es kann alternativ oder zusätzlich auch ein Wirkstoff und/oder ein Geruchsstoff, z.B. gegen schlechten Atem, freigesetzt werden. Dabei ist die Variation der Fig. 4g nicht zwingend auf ein dreidimensionales Objekt, wie eine Kugel, festgelegt, sondern es kann auch eine Beschichtung oder dergleichen sein.

Fig. 4h zeigt eine weitere Variante zur Anbringung eines Zungen-Ankerpunktes 9 an eine Überbrückung 10. Hier in der Variante als ein Clip oder Manschette 12. Der Clip ist einseitig geöffnet und kann durch Aufspreizen über die Überbrückung geführt und sodann verklemmt werden. Die Manschette 12 kann durch Lösen der Überbrückung von endständiger Seite bzw. von der Seite der mechanischen Verbindung mit dem Grundkörper über diese übergestreift werden. Auch Bajonett-Verbindungen sind im Rahmen der vorliegenden Erfindung zur Verbindung des Zungen-Ankerpunkts mit der Überbrückung im Rahmen der vorliegenden Erfindung möglich.

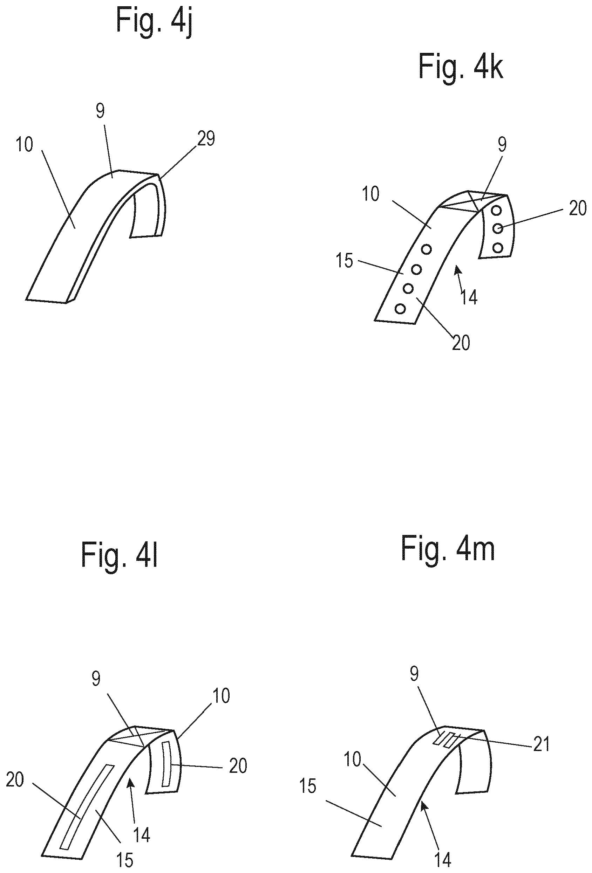

Fig. 4j zeigt eine Variante einer Überbrückung 10 mit einem Zungen-Ankerpunkt 9, wobei der Zungen-Ankerpunkt sich analog zu Fig. 4a lediglich aufgrund seiner Position mittig an der Überbrückung 10 von benachbarten Bereichen unterscheidet. Die Überbrückung kann eine ein- oder mehrlagige Beschichtung 29 oder eine Laminierung aufweisen. Vorzugsweise kann die Beschichtung oder Laminierung kann einen Wirkstoff und/oder einen Geschmacksstoff und/oder einen Geruchsstoff aufweisen, welcher insbesondere zumindest teilweise in Speichel löslich und damit freisetzbar ist. Die Überbrückung 10 kann alternativ auch als Hohlelement ausgebildet sein.

Eine Oberflächenaufrauhung oder -glättung im Bereich des Zungen-Ankerpunkts 9, also mittigen Bereich der Überbrückung 10, kann auch durch chemische oder mechanische Bearbeitung wie Ätzen und/oder Schleifen erreicht werden.

Auch die zum Zungen-Ankerpunkt 9 benachbarten Bereiche 15 können verschiedene Depots, Beschichtungen, An- und/oder Einlagerungen oder dergleichen aufweisen. So können z.B. Wirk- oder Geschmacksstoffe in den benachbarten Bereichen angeordnet sein. Dies ist in den Fig. 4k und 4l dargestellt. Ein entsprechendes Depot 20 kann punktförmig oder als Streifen ausgebildet sein. In Fig. 4k und 4l ist der Zungen-Ankerpunkt 9 eine haptisch-differente Fläche, z.B. als Lackschicht, ausgebildet.

In Fig. 4m weist die Überbrückung 10 zumindest im Bereich des Zungen-Ankerpunkts 9 einer oder mehrere Streifen 21 an der konkaven Seite der Überbrückung auf. Der oder die Streifen 21 weist bzw. weisen eine andere Oberflächentextur auf als die dazu benachbarten Flächen. Der oder die Streifen 21 können sich auch über die gesamte Länge der Überbrückung erstrecken.

Die Überbrückung 10 der Fig. 4n weist zumindest im Bereich des Zungen-Ankerpunkts 9 Öffnungen, insbesondere einer oder mehrere parallel zur Längserstreckung der Überbrückung 10 erstreckende Schlitze 22.

In Fig. 4p weist die Überbrückung 10 eine von der konkaven Seite hervorstehende Zungen-Ansaugvorrichtung 23 auf, welche ebenfalls einen Teil eines Zungen-Ankerpunkts 9 bilden kann.

Alternativ kann auch eine Zungenauflage- oder Zungenhaltevorrichtung 24, wie in Fig. 4q dargestellt, als Teil eines Zungen-Ankerpunkts 9 ausgebildet sein.

In Fig. 4s kann eine Überbrückung 10 im Bereich des Zungenankerpunkts 9 eine andere Oberflächenflächenenergie, eine andere Hydrophobie/Hydrophilie oder eine andere elektrische Leitfähigkeit, insbesondere eine Metallfläche und eine Polymerfläche wie die jeweiligen benachbarten Flächen 15.

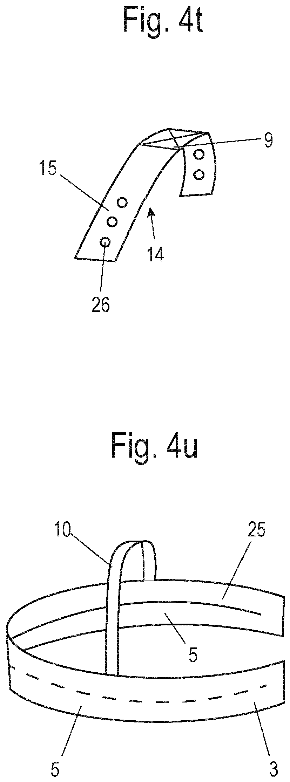

In Fig. 4t weist die Überbrückung in einem oder in beiden gegenüber dem Zungen-Ankerpunkt 9 benachbarten Bereichen Sensoren 26 und/oder Aktuatoren angeordnet.

Wie sich aus den vorgenannten Figuren ergibt, ist ein Zungen-Ankerpunkt 9 im Sinne der vorliegenden Anmeldung insbesondere als Bereich und nicht nur als Punkt im streng-mathematischen Sinne zu verstehen.

Wie in Fig. 4u dargestellt, kann der Grundkörper 3 ein Zahnaufhellungsstreifen 25 angeordnet sein. Dieser kann insbesondere austauschbar in dem Grundkörper 3 angeordnet sein. Besonders bevorzugt kann die Außenwandung 5 oder die Innenwandung 6 eine auf der zahninnenliegenden Seite angeordnete Ausnehmung aufweisen, welche die Aufnahme eines entsprechenden Zahnaufhellungsstreifens 25 erlaubt.

Die in Fig. 4a -4u dargestellten Varianten sind nur einige Beispiele für die Ausführung des Gegenstands der Erfindung. Es sind zahlreiche weitere Varianten im Rahmen der vorliegenden Erfindung möglich.

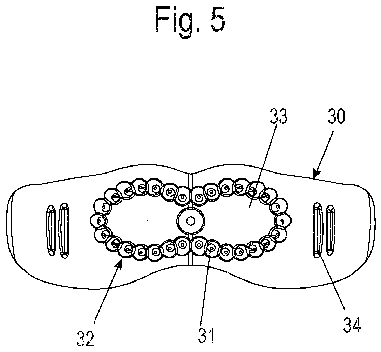

Fig. 5 zeigt eine spezielle Ausgestaltung einer Außenwandung 5 der Grundform 3 als Schildsegment 30. Das Schildsegment 30 kann zwischen Zahnreihe und Lippen, zum bereichsweisen Überdecken einer Zahnreihe, insbesondere der Zahnreihen des Ober- und Unterkiefers, angeordnet sein. Das Schildsegment 30 weist vorteilhaft auf der zahnabgewandten Seite zumindest einen Vorsprung, insbesondere eine Anordnung mehrerer Vorsprünge 31, zur Lippenstimulation aufweist.

Die Vorsprünge 31 können vorzugsweise eine Höhe von zumindest 0.8 mm, vorzugsweise grösser 1.0 mm aufweisen. Ebenfalls vorzugsweise können die Vorsprünge zylindrisch, kalotten- oder kegelstumpfförmig ausgebildet sein.

Auch die Vorsprünge 31 des Schildsegments können zur Überwachung des fortschreitenden Trainingserfolgs und/oder des Gesundheitszustands im Mund Sensorelemente aufweisen. So können die Vorsprünge 31 derart angeordnet sein, dass sie eine Aufnahme 32 mit einem Aufnahmebereich 33 ausbilden, in welchen ein austauschbares Sensorelement oder ein Sensor anordenbar ist.

An gleicher Stelle können alternativ oder zusätzlich auch Aktuatoren z.B. zur zusätzlichen elektrischen oder mechanischen Lippenstimulanz eingesetzt werden. Randseitig zu den im Mittelsegment angeordneten Vorsprüngen 31 weist das Schildsegment 30 Schlitze 34 zum Speichelabfluss auf.

### Bezugszeichen

- 1: Aligner
- 2: Zahneinzelkonturen
- 3: U-förmige Grundform
- 4: Zahnzwischenräume
- 5: Außenwandung
- 6: Innenwandung
- 7: Schalenform
- 8: Innenraum
- 9: Zungen-Ankerpunkt
- 10: Überbrückung
- 11: Vorsprung
- 12: Manschette
- 13: Kugel
- 14: konkave Seite
- 15: benachbarte Bereiche
- 16: faden- oder streifenartig abstehende Elementen 16
- 17: flexible Nadeln
- 18: konkave Ausbuchtung/Nase
- 19: Sockel
- 20: Depot
- 21: Streifen
- 22: Schlitze
- 23: Zungen-Ansaugvorrichtung
- 24: Zungenauflage- oder Zungenhaltevorrichtung
- 25: Zahnaufhellungsstreifens
- 26: Sensoren
- 27: Schildsegment
- 28: Vorsprung
- 29: Beschichtung
- 30: Schildsegment
- 31: Vorsprung
- 32: Aufnahme
- 33: Aufnahmebereich
- 34: Schlitze

## Patentansprüche

1. Aligner (1) zum Ausrichten von Zähnen eines Gebisses, wobei der Aligner (1) eine Grundform (3) zur Vorgabe einer bevorzugten Zahnstellung aufweist, wobei die Grundform (3) eine U-Form aufweist, welche einen zur U-Form-komplementären bogenförmigen Innenraum (8) begrenzt, wobei der Aligner einen von der Grundform (3) in Richtung des Innenraums (8) gerichteten Zungen-Ankerpunkt (9) zur Positionierung im bestimmungsgemäßen Zustand nahe dem Gaumendach einer Mundhöhle aufweist,
wobei der Zungen-Ankerpunkt (9) als Teil eines Vorsprungs (11) ausgebildet ist, welcher von einer Innenwandung (6) des Aligners zur lingualen Bedeckung einer Zahnreihe in Richtung des Innenraums (8) hervorsteht oder
wobei der Zungen-Ankerpunkt (9) als Teil einer Überbrückung (10) ausgebildet ist, welche von dem ersten Schenkel der U-Form zum zweiten Schenkel der U-Form verläuft und somit den Innenraum (8) überbrückt,
wobei der Zungen-Ankerpunkt (9) eine geometrische Figur aufweist, welche gegenüber einem Verbindungsbereich des Vorsprungs (11) und/oder der Überbrückung (10) mit der Grundform (3) zumindest teilweise hervorsteht,
**dadurch gekennzeichnet, dass** der Zungen-Ankerpunkt gegenüber dem Vorsprung oder der Überbrückung lösbar und austauschbar angeordnet ist.

2. Aligner nach Anspruch 1, **dadurch gekennzeichnet, dass** die Grundform (3), die Innenwandung (6) zur lingualen Bedeckung einer Zahnreihe aufweist.

3. Aligner nach Anspruch 1 oder 2, **dadurch gekennzeichnet, dass** der Aligner (1), insbesondere die Grundform (3), eine Außenwandung (5) zur labialen Überdeckung einer Zahnreihe aufweist.

4. Aligner nach einem der vorhergehenden Ansprüche, **dadurch gekennzeichnet, dass** der Zungen-Ankerpunkt gegenüber dem Vorsprung oder der Überbrückung verrastbar angeordnet ist.

5. Aligner nach einem der vorhergehenden Ansprüche, **dadurch gekennzeichnet, dass** der Aligner (1), insbesondere die Grundform (3), Zahneinzelkonturen (2) mit einer vordefinierten Zahnstellung und mit zumindest teilweise buccalen Überdeckungsbereichen von einzelnen Zähnen einer Zahnreihe aufweist.

6. Aligner nach einem der vorhergehenden Ansprüche, **dadurch gekennzeichnet, dass** der Zungen-Ankerpunkt (9) einen runden Körper, insbesondere eine Kugel (13) oder einen Ovoid, aufweist.

7. Aligner nach einem der vorhergehenden Ansprüche, **dadurch gekennzeichnet, dass** zumindest der Vorsprung (11) oder die Überbrückung (10) gegenüber der Grundform (3) lösbar, vorzugsweise austauschbar, insbesondere verrastbar, angeordnet ist.

8. Aligner nach einem der vorhergehenden Ansprüche, **dadurch gekennzeichnet, dass** der Zungen-Ankerpunkt (9), gegenüber benachbarten Bereichen (15) der Überbrückung (10) oder des Vorsprungs (11), eine differente Oberflächenhaptik, vorzugsweise eine verschiedene Rauhigkeit und/oder Weichheit des Materials, aufweist.

9. Aligner nach einem der vorhergehenden Ansprüche, **dadurch gekennzeichnet, dass** der Zungen-Ankerpunkt (9) eine in Speichel zumindest teilweise lösliche chemische Verbindung, insbesondere einen Geschmacksstoff oder einen Wirkstoff, aufweist.

10. Aligner nach einem der vorhergehenden Ansprüche, **dadurch gekennzeichnet, dass** im Bereich des Zungen-Ankerpunkts (9) Öffnungen, insbesondere Schlitze (22), vorgesehen sind.

11. Aligner nach einem der vorhergehenden Ansprüche, **dadurch gekennzeichnet, dass** im Bereich des Zungen-Ankerpunkts (9) ein Material aus einer anderen chemischen Zusammensetzung angeordnet ist, oder der Bereich oder der Zungen-Ankerpunkt (9) insbesondere aus einer anderen Materialklasse besteht, als die benachbarten Bereiche (15) des Vorsprungs (11) und/oder der Überbrückung (10).

12. Aligner nach einem der vorhergehenden Ansprüche, **dadurch gekennzeichnet, dass** der Zungen-Ankerpunkt (9) bewegliche Elemente aufweist, welche durch eine Zunge bewegbar sind.

13. Aligner nach einem der vorhergehenden Ansprüche, **dadurch gekennzeichnet, dass** der Aligner (1) ein Schildsegment (30) zum bereichsweisen Überdecken einer Zahnreihe, insbesondere der Zahnreihen des Ober- und Unterkiefers, aufweist wobei das Schildsegment auf der zahnabgewandten Seite zumindest einen Vorsprung (31), insbesondere eine Anordnung mehrerer Vorsprünge, zur Lippenstimulation aufweist.

14. Aligner nach einem der vorhergehenden Ansprüche, **dadurch gekennzeichnet, dass** der Aligner (1) Sensoren (26) zur Ermittlung deiner physikalischen und/oder chemischen Größe im Speichel und/oder im Mundraum aufweist, wobei die Sensoren besonders bevorzugt Teil des Vorsprungs (11) oder der Überbrückung (10) sind.

15. Aligner nach einem der vorhergehenden Ansprüche, **dadurch gekennzeichnet, dass** der Aligner (1) Aktuatoren, insbesondere Elektroden, zur Erzeugung und/oder Übermittlung von Stimulanzsignalen aufweist, wobei die Aktuatoren besonders bevorzugt Teil des Vorsprungs (11) oder der Überbrückung (10) sind.

## Claims

1. Aligner (1) for aligning the teeth of a dentition, wherein the aligner (1) comprises a base form (3) for defining a preferred tooth position, wherein the base form (3) has a U-shape that defines an arcuate interior space (8) complementary to the U-shape, wherein the aligner comprises a tongue anchor point (9) extending from the base form (3) directed toward the interior space (8) for positioning, in the intended state, near the roof of the mouth,
wherein the tongue anchor point (9) is formed as part of a projection (11) which extends from an inner wall (6) of the aligner toward the interior space (8) for lingual coverage of a row of teeth, or
wherein the tongue anchor point (9) is formed as part of a bridge (10) that extends from the first leg of the U-shape to the second leg of the U-shape and thus bridges the interior space (8),
wherein the tongue anchor point (9) has a geometric shape that protrudes at least partially relative to a connection area of the projection (11) and/or the bridge (10) with the base form (3), **characterized in that** the tongue anchor point is arranged to be detachable and replaceable relative to the projection or the bridge.

2. Aligner according to claim 1, **characterized in that** the base form (3) comprises the inner wall (6) for lingual coverage of a row of teeth.

3. Aligner according to claim 1 or 2, **characterized in that** the aligner (1), in particular the base form (3), comprises an outer wall (5) for labial coverage of a row of teeth.

4. Aligner according to one of the preceding claims, **characterized in that** the tongue anchor point is arranged to be lockable relative to the projection or the bridge.

5. Aligner according to one of the preceding claims, **characterized in that** the aligner (1), in particular the base form (3), comprises individual tooth contours (2) with a predefined tooth position and with at least partially buccal covering areas of individual teeth in a row of teeth.

6. Aligner according to one of the preceding claims, **characterized in that** the tongue anchor point (9) comprises a round body, in particular a sphere (13) or an ovoid.

7. Aligner according to one of the preceding claims, **characterized in that** at least the projection (11) or the bridge (10) is arranged in a detachable, preferably interchangeable, and in particular lockable manner relative to the base form (3).

8. Aligner according to one of the preceding claims, **characterized in that** the tongue anchor point (9) has a different surface texture, preferably a different roughness and/or softness of the material, compared to adjacent areas (15) of the bridge (10) or the projection (11).

9. Aligner according to one of the preceding claims, **characterized in that** the tongue anchor point (9) comprises a chemical compound at least partially soluble in saliva, in particular a flavoring agent or an active ingredient.

10. Aligner according to one of the preceding claims, **characterized in that** openings, in particular slits (22), are provided in the region of the tongue anchor point (9).

11. Aligner according to one of the preceding claims, **characterized in that** a material of a different chemical composition is arranged in the region of the tongue anchor point (9), or the region or the tongue anchor point (9) consists, in particular, of a different material class than the adjacent regions (15) of the projection (11) and/or the bridge (10).

12. Aligner according to one of the preceding claims, **characterized in that** the tongue anchor point (9) comprises movable elements that can be moved by a tongue.

13. Aligner according to one of the preceding claims, **characterized in that** the aligner (1) comprises a shield segment (30) for covering a row of teeth in sections, in particular the rows of teeth of the upper and lower jaws, wherein the shield segment comprises, on the side facing away from the teeth, at least one projection (31), in particular an arrangement of multiple projections, for lip stimulation.

14. Aligner according to one of the preceding claims, **characterized in that** the aligner (1) comprises sensors (26) for determining a physical and/or chemical parameter in saliva and/or in the oral cavity, wherein the sensors are particularly preferably part of the projection (11) or the bridge (10).

15. Aligner according to one of the preceding claims, **characterized in that** the aligner (1) comprises actuators, in particular electrodes, for generating and/or transmitting stimulation signals, wherein the actuators are particularly preferably part of the projection (11) or the bridge (10).

## Revendications

1. Aligneur (1) destiné à redresser des dents d'une arcade dentaire, l'aligneur (1) comportant une forme de base (3) destinée à déterminer une position préférée des dents, laquelle forme de base (3) présente une forme de U qui délimite un espace intérieur (8) en arc de cercle complémentaire de la forme en U, l'aligneur comportant un point d'ancrage lingual (9) orienté de la forme de base (3) vers l'espace intérieur (8) et servant au positionnement dans l'état adéquat près de la voûte du palais d'une cavité buccale,
dans lequel le point d'ancrage lingual (9) est conformé comme une partie d'une saillie (11) qui dépasse d'une paroi intérieure (6) de l'aligneur en direction de l'espace intérieur (8) pour couvrir une rangée de dents du côté lingual ou dans lequel le point d'ancrage lingual (9) est conçu comme une partie d'un pont (10) qui va du premier bras de la forme en U au deuxième bras de la forme en U et enjambe ainsi l'espace intérieur (8),
dans lequel le point d'ancrage lingual (9) présente une figure géométrique qui dépasse au moins en partie par rapport à une zone de liaison de la saillie (11) et/ou du pont (10) avec la forme de base (3),
**caractérisé en ce que** le point d'ancrage lingual est disposé vis-à-vis de la saillie ou du pont de façon amovible et interchangeable.

2. Aligneur selon la revendication 1, **caractérisé en ce que** la forme de base (3) présente la paroi intérieure (6) pour la couverture du côté lingual d'une rangée de dents.

3. Aligneur selon la revendication 1 ou 2, **caractérisé en ce que** l'aligneur (1), en particulier la forme de base (3), comporte une paroi extérieure (5) pour la couverture du côté vestibulaire d'une rangée de dents.

4. Aligneur selon l'une des revendications précédentes, **caractérisé en ce que** le point d'ancrage lingual est disposé vis-à-vis de la saillie ou du pont de façon à pouvoir être emboîté.

5. Aligneur selon l'une des revendications précédentes, **caractérisé en ce que** l'aligneur (1), en particulier la forme de base (3), comporte des contours de dents unitaires (2) ayant une position de dent prédéfinie et des zones de chevauchement buccal, au moins en partie, de certaines dents d'une rangée de dents.

6. Aligneur selon l'une des revendications précédentes, **caractérisé en ce que** le point d'ancrage lingual (9) comporte un corps rond, en particulier une bille (13) ou un ovoïde.

7. Aligneur selon l'une des revendications précédentes, **caractérisé en ce que** la saillie (11) ou le pont (10), au moins, est disposé vis-à-vis de la forme de base (3) de façon amovible, de préférence interchangeable, en particulier emboîtable.

8. Aligneur selon l'une des revendications précédentes, **caractérisé en ce que** le point d'ancrage lingual (9) présente, par rapport aux régions voisines (15) du pont (10) ou de la saillie (11), une texture de surface différente, de préférence une rugosité et/ou une souplesse du matériau différente.

9. Aligneur selon l'une des revendications précédentes, **caractérisé en ce que** le point d'ancrage lingual (9) comporte un composé chimique au moins partiellement soluble dans la salive, en particulier un arôme ou un principe actif.

10. Aligneur selon l'une des revendications précédentes, **caractérisé en ce que** des ouvertures, en particulier des fentes (22), sont prévues dans la région du point d'ancrage lingual (9).

11. Aligneur selon l'une des revendications précédentes, **caractérisé en ce qu'**est disposé dans la région du point d'ancrage lingual (9) un matériau d'une autre composition chimique, ou la région ou le point d'ancrage lingual (9) se compose en particulier d'un matériau d'une autre classe que les régions voisines (15) de la saillie (11) et/ou du pont (10).

12. Aligneur selon l'une des revendications précédentes, **caractérisé en ce que** le point d'ancrage lingual (9) comporte des éléments mobiles qui peuvent être déplacés par la langue.

13. Aligneur selon l'une des revendications précédentes, **caractérisé en ce que** l'aligneur (1) comporte un segment de bouclier (30) pour la couverture partielle d'une rangée de dents, en particulier des rangées de dents du maxillaire et de la mandibule, le segment de bouclier comportant au moins une saillie (31) sur la face opposée aux dents, en particulier une disposition de plusieurs saillies pour la stimulation des lèvres.

14. Aligneur selon l'une des revendications précédentes, **caractérisé en ce que** l'aligneur (1) comporte des capteurs (26) destinés à déterminer une grandeur physique et/ou chimique dans la salive et/ou dans la cavité buccale, lesquels capteurs font de préférence partie de la saillie (11) ou du pont (10).

15. Aligneur selon l'une des revendications précédentes, **caractérisé en ce que** l'aligneur (1) comporte des actionneurs, en particulier des électrodes, pour produire et/ou transmettre des signaux de stimulation, lesquels actionneurs font de préférence partie de la saillie (11) ou du pont (10).
